# EUROPEAN PATENT APPLICATION

(11) **EP 3 175 861 A1**
(43) Date of publication of application: **07.06.2017**
(21) Application number: 14898399.2
(22) Date of filing: 23.10.2014
(51) Int. Cl.: A61K 36/8988, A61K 36/73, A61K 36/725, A61K 36/537, A61K 36/428, A61K 36/258, A61K 36/236, A61K 36/185, A61K 36/16, A61K 35/56, A61K 31/12, A61K 9/20, A61P 9/00

(54) **PHARMACEUTICAL COMPOSITION FOR TREATING CARDIOVASCULAR OR CEREBROVASCULAR DISEASES AND PREPARATION METHOD THEREFOR**

(30) Priority: 31.07.2014 CN 201410374688
(71) Applicant: Du, Zhizheng, Beijing 100162 (CN); Tao, Aftermath, Beijing 100162 (CN)
(72) Inventor: Du, Zhizheng, Beijing 100162 (CN); Tao, Aftermath, Beijing 100162 (CN)
(74) Representative: Hryszkiewicz, Danuta
(86) International application number: PCT/CN2014/089347
(87) International publication number: WO 2016/015391

(57) **Abstract**

A pharmaceutical composition for treating cardiovascular or cerebrovascular diseases. By weight parts, the components thereof are ginkgo flavanoids 100-170 pts., common jujube extract 40-100 pts., snakegourd fruit extract 100-200 pts., radix codonopsis saponins 30-60 pts., radix panacis quinquefolii saponins 50-100 pts., astragalosides 40-70 pts., lepirudin 20-50 pts., coenzyme Q10 3-10 pts., longan extract 40-100 pts., modified starch 20-50 pts., carthamin 100-150 pts., peach seed extract 100-150 pts., gastrodin 30-50 pts., salvianolic acids 120-200 pts., ligusticum chuanxiong extract 40-90 pts., panax notoginsenosides 100-150 pts., sesamin 0-50 pts., astaxanthin 0-50 pts.. The pharmaceutical composition may used for treating hypertension, hyperlipemia, insomnia, coronary heart disease, sequelae of cerebral stroke, etc.

## Description

The invention relates to the technical field of Chinese herbs, and more particularly to a pharmaceutical composition for use in treating cardio-cerebrovascular disease and a method for preparing the same.

At present, there are 1 billion people worldwide suffering from hypertension, and almost 20 million people died from hypertension and cardio-cerebrovascular diseases.

According to the report of cardio-cerebrovascular diseases in China in 2013, the number of patients suffered from the cardio-cerebrovascular diseases (coronary heart disease, stroke, heart failure and hypertension) is 290 million, including 266 million people with hypertension, 7 million people with stroke, 2.5 million people with myocardial infarction, 4.5 million people with heart failure, 5 million people with pulmonary heart disease, 2.5 million people with rheumatic heart disease, 200 million people with congenital heart disease. Two out of every 10 adults have cardio-cerebrovascular diseases, and 9590 people died from the cardio-cerebrovascular diseases each day, accounting for 51 % of the total cause of death in China, ranking the first in various diseases. Both the incidence and the mortality of cardio-cerebrovascular diseases are the first in the world.

China's cardio-cerebrovascular diseases report shows that the incidence of stroke is 4-5 times the number of myocardial infarction, and more common in male than in female. the prevalence of dementia in urban residents older than 55 years of age was 4.7%, and 7.8% in people older than 65 years of age.

Hypertension is a major risk factor for stroke and coronary heart disease. Almost 50% of people having normal blood pressure suffer from hypertension within one decade. The prevalence rate of hypertension in adults over 18 years of age was 18.8%, or high than 25% in some areas, or even higher than 30% in northern parts.

Risk factors of the cardio-cerebrovascular diseases trend to be obvious. As the prevalence rate is rising, the cardio-cerebrovascular diseases become a major public health problem and an important cause of poverty. Thus, it is an urgent to solve the problem of cardio-cerebrovascular diseases.

In view of the above-described problems, it is one objective of the invention to provide a pharmaceutical composition for use in treating cardio-cerebrovascular disease. The cardio-cerebrovascular disease is one or several of hypertension, hyperlidemia, heart diseases, rheumatic heart diseases, both right and left atrial enlargement, serious ischemic heart diseases, angina pectoris, coronary arteriosclerosis, coronary atherosclerosis, shortness of breath, myocardial infarction, cerebral infarction, thromboembolisms, sequela of cerebrovascular disease, poststroke syndrome, and apoplectic hemiplegia.

It is another objective of the invention to provide a method for preparing the pharmaceutical composition for use in treating cardio-cerebrovascular disease.

A pharmaceutical composition for use in treating cardio-cerebrovascular disease, comprises: 100-170 parts by weight of a ginkgo flavone, 40-100 parts by weight of a jujube extract, 100-200 parts by weight of an extract of peel of *Trichosanthes kirilowii*, 30-60 parts by weight of a saponin of *Codonopsis,* 50-100 parts by weight of a saponin of American ginseng, 40-70 parts by weight of a saponin of *Radix astragalu*, 20-50 parts by weight of hirudin, 3-10 parts by weight of a coenzyme Q10, 40-100 parts by weight of a longan extract, 20-50 parts by weight of a modified starch, 100-150 parts by weight of a carthamin, 100-150 parts by weight of an extract of peach kernel, 30-50 parts by weight of a gastordin, 120-200 parts by weight of a salvianolic acid, 40-90 parts by weight of an extract of *Ligusticum chuanxiong,* 100-150 parts by weight of a saponin of *Panax notoginseng,* 0-50 parts by weight of a black sesamin, 0-50 parts by weight of an astaxanthin.

Preferably, the ginkgo flavone has a purity of equal to or larger than 98%, and a dose of the ginkgo flavone is 150-170 parts by weight; a dose of the jujube extract is 50-80 parts by weight; and a dose of the extract of the peel of *Trichosanthes kirilowii* is 130-160 parts by weight.

Preferably, a purity of the saponin of *Codonopsis* is equal to or larger than 80%, and a dose of the saponin of *Codonopsis* is 40-50 parts by weight; a purity of the saponin of American ginseng is larger than 80%, and a dose of the saponin of American ginseng is 60-70 parts by weight; a purity of the saponin of *Radix astragalus* is equal to or larger than 80%, and a dose of the saponin of American ginseng is 50-60 parts by weight.

Preferably, a purity of the hirudin is equal to or larger than 98%, and a dose of the hirudin is 25-50 parts by weight; a purity of the coenzyme Q10 is equal to or larger than 80%, and a dose of the coenzyme Q10 is 4-8 parts by weight; and a dose of the longan extract is 50-80 parts by weight.

Preferably, a dose of the modified starch is 30-40 parts by weight; a purity of the carthamin is equal to or larger than 80%, and a dose of the carthamin is 130-150 parts by weight; and a dose of the extract of peach kernel is 100-120 parts by weight.

Preferably, a purity of the gastordin is equal to or larger than 98%, and a dose of the gastordin is 35-50 parts by weight; a purity of the salvianolic acid is equal to or larger than 98%, and a dose of the salvianolic acid is 140-150 parts by weight; and a dose of the extract of *Ligusticum chuanxiong* is 50-70 parts by weight.

Preferably, a purity of the saponin of *Panax notoginseng* is equal to or larger than 98%, and a dose of the saponin of *Panax notoginseng* is 120-140 parts by weight.

Preferably, a purity of the black sesamin is equal to or larger than 98%, and a dose of the black sesamin is 30-50 parts by weight.

Preferably, a purity of the astaxanthin is equal to or larger than 10%, and a dose of the astaxanthin is 30-50 parts by weight.

A method for preparing the pharmaceutical composition comprises: 1) collecting raw materials for the pharmaceutical composition according to a formula, and uniformly mixing the raw materials in a dish to yield a mixture; 2) tableting the mixture, polishing resulting tablets, and sterilizing the tablets to yield a product. Sterilization is conducted at a temperature of 90-100°C and a time of 20-30 min.

A pharmaceutical composition for use in treating cardio-cerebrovascular disease, comprising: 100-170 parts by weight of a ginkgo flavone, 40-100 parts by weight of a jujube extract, 100-200 parts by weight of an extract of peel of *Trichosanthes kirilowii*, 30-60 parts by weight of a saponin of *Codonopsis,* 50-100 parts by weight of a saponin of American ginseng, 40-70 parts by weight of a saponin of *Radix astragalu*, 20-50 parts by weight of hirudin, 3-10 parts by weight of a coenzyme Q10, 40-100 parts by weight of a longan extract, 100-150 parts by weight of a carthamin, 100-150 parts by weight of an extract of peach kernel, 0-50 parts by weight of a gastordin, 120-200 parts by weight of a salvianolic acid, 40-90 parts by weight of an extract of *Ligusticum chuanxiong,* 100-150 parts by weight of a saponin of *Panax notoginseng,* 0-50 parts by weight of a black sesamin, and 0-50 parts by weight of an astaxanthin.

Preferably, the pharmaceutical composition further comprises 20-50 parts by weight of a modified starch; and preferably 30-40 parts by weight of the modified starch.

Preferably, a dose of the jujube extract is 50-80 parts by weight.

Preferably, a purity of the saponin of *Codonopsis* is equal to or larger than 80%, and a dose of the saponin of *Codonopsis* is 40-50 parts by weight.

Preferably, a purity of the saponin of American ginseng is larger than 80%, and a dose of the saponin of American ginseng is 60-70 parts by weight.

Preferably, a purity of the saponin of *Radix astragalus* is equal to or larger than 80%, and a dose of the saponin of *Radix astragalus* is 50-60 parts by weight.

Preferably, a purity of the hirudin is equal to or larger than 98%, and a dose of the hirudin is 25-50 parts by weight.

Preferably, the coenzyme Q10 is a liposoluble coenzyme Q10 having a purity of being equal to or larger than 80%, and a dose of the coenzyme Q10 is 4-8 parts by weight.

Preferably, a dose of the longan extract is 50-80 parts by weight.

Preferably, a purity of the salvianolic acid is equal to or larger than 98%, and a dose of the salvianolic acid is 140-150 parts by weight.

Preferably, a purity of the ginkgo flavone is equal to or larger than 98%, and a dose of the ginkgo flavone is150-170 parts by weight; and a dose of the extract of peel of *Trichosanthes kirilowii* is 130-160 parts by weight.

Preferably, a purity of the carthamin is equal to or larger than 80%, and a dose of the carthamin is 130-150 parts by weight; and a dose of the extract of peach kernel is 100-120 parts by weight.

Preferably, a purity of the gastordin is equal to or larger than 98%, and a dose of the gastordin is 30-50 parts by weight, preferably 35-50 parts by weight; and a dose of the extract of *Ligusticum chuanxiong* is 50-70 parts by weight.

Preferably, a purity of the saponin of *Panax notoginseng* is equal to or larger than 98%, and a dose of the saponin of *Panax notoginseng* is 120-140 parts by weight.

Preferably, a purity of the black sesamin is equal to or larger than 98%, and a dose of the black sesamin is 30-50 parts by weight.

Preferably, a purity of the astaxanthin is equal to or larger than 10%, and a dose of the astaxanthin is 30-50 parts by weight.

Preferably, the composition comprises: 150 parts by weight of the ginkgo flavone, 80 parts by weight of the jujube extract, 130 parts by weight of the extract of peel of *Trichosanthes kirilowii*, 50 parts by weight of the saponin of *Codonopsis*, 60 parts by weight of the saponin of American ginseng, 50 parts by weight of the saponin of *Radix astragalu*, 50 parts by weight of hirudin, 4 parts by weight of the coenzyme Q10, 70 parts by weight of the longan extract, 130 parts by weight of the carthamin, 100 parts by weight of the extract of peach kernel, 50 parts by weight of the gastordin, 170 parts by weight of the salvianolic acid, 60 parts by weight of the extract of *Ligusticum chuanxiong,* 140 parts by weight of saponin of *Panax notoginseng,* 50 parts by weight of the black sesamin, and 36 parts by weight of the astaxanthin.

A method for preparing the pharmaceutical composition comprises: 1) collecting raw materials for the pharmaceutical composition according to a formula, and uniformly mixing the raw materials in a dish to yield a mixture; 2) tableting the mixture, polishing resulting tablets, and sterilizing the tablets to yield a product. Sterilization is conducted at a temperature of 90-100°C and a time of 20-30 min.

Use of the pharmaceutical composition for treating hypertension, hyperlidemia, heart diseases, rheumatic heart diseases, both right and left atrial enlargement, serious ischemic heart diseases, angina pectoris, coronary arteriosclerosis, coronary atherosclerosis, shortness of breath, myocardial infarction, cerebral infarction, thromboembolisms, sequela of cerebrovascular disease, poststroke syndrome, or apoplectic hemiplegia.

Preferably, the pharmaceutical composition is used combining with use of a pharmaceutical composition of *Fructus liquidambaris.*

For further illustrating the invention, experiments detailing a pharmaceutical composition for use in treating cardio-cerebrovascular disease and a method for preparing the same are described hereinbelow combined with the drawings.

In a specific embodiment of the invention, a pharmaceutical composition for use in treating cardio-cerebrovascular disease comprises: 100-170 parts by weight of a ginkgo flavone, 40-100 parts by weight of a jujube extract, 100-200 parts by weight of an extract of peel of *Trichosanthes kirilowii*, 30-60 parts by weight of a saponin of *Codonopsis,* 50-100 parts by weight of a saponin of American ginseng, 40-70 parts by weight of a saponin of *Radix astragalu*, 20-50 parts by weight of hirudin, 3-10 parts by weight of a coenzyme Q10, 40-100 parts by weight of a longan extract, 100-150 parts by weight of a carthamin, 100-150 parts by weight of an extract of peach kernel, 0-50 parts by weight of a gastordin, 120-200 parts by weight of a salvianolic acid, 40-90 parts by weight of an extract of *Ligusticum chuanxiong,* 100-150 parts by weight of a saponin of *Panax notoginseng,* 0-50 parts by weight of a black sesamin, and 0-50 parts by weight of an astaxanthin.

Main principles for the pharmaceutical composition for treating the cardio-cerebrovascular diseases are explained as follows:

The ginkgo flavone, the jujube extract, the saponin of *Codonopsis*, saponin of American ginseng, the saponin of *Radix astragalus,* the hirudin, the longan extract, the extract of peach kernel, the gastordin, the salvianolic acid, the saponin of *Panax notoginseng,* and the black sesamin contain natural *Calculus bovis,* isoflavone, saponins, phospholipids, and multiple enzymes and function in invigorating qi, fast and effectively decomposing and dissolving lipids, thrombus, and other blood mass and precipitates.

It is demonstrated from experiments that after administration of the pharmaceutical composition by patients, the phospholipids, flavone, isoflavone, saponins, and compound enzymes of small molecular contained in the pharmaceutical composition are quickly permeated into atherosclerotic plaques and decompose lipid masses in the blood by breaking walls of lipid cells to gradually remove the lipids, decompose the plaques, expands and soften the blood vessels. As the expansion and the elasticity of the blood vessels are recovered, the resistance of the blood vessels is reduced, the flow velocity of the blood is facilitated, the pressure is reduced, the heartbeat and the breath become normal, an average increase of the proteoliposome is 33%, and an average decrease of triglyceride is 28%, a removal rate of the thrombus exceeds 98%. The blood supply for the blood vessels of the heart and brain, and hypertension and hyperlidemia almost disappear. The mechanism of the pharmaceutical composition is as follows:
1) when combining with the thrombin, the pharmaceutical composition inhibits enzymolysis of the thrombin and the combining action of the thrombomodulin of endothelial cells;
2) coagulation and release of platelets induced by the thrombin are prevented;
3) fibroblast proliferation and smooth muscle cells contraction stimulated by the thrombin are inhibited; and
4) contraction of injured blood vessels acted by the thrombin is inhibited, thus presenting excellent anti-coagulation effect.

The action of the pharmaceutical composition is specifically explained in five steps:
First, invigorating qi and promoting blood circulation. The saponin of *Codonopsis,* the saponin of American ginseng, the saponin of *Radix astragalus*, the longan extract, the hirudin, the extract of *Eupolyphaga,* and the extract of *Scorpio* have the effect of invigorating qi and promoting blood circulation.
Second, soothing liver and clearing blood. The carthamin, the extract of peach kernel, the extract of peel of *Trichosanthes kirilowii,* and the salvianolic acid are able to sooth liver, clear blood, and decompose lipids and cholesterol, thus removing a polluting source of blood.
Third, invigorating liver and kidney and filtering blood. Extracts of the gastordin, the black sesamin, and the astaxanthin are able to nourish the kidney essence and quickly clear blood vessels.
Fourth, enhancing heart for pumping blood. The ginkgo flavone, the coenzyme Q10 (liposoluble), the saponin of *Panax notoginseng,* the extract of *Ligusticum chuanxiong* are able to soften the blood vessels, recover the elasticity of cardiac muscles, and protect the heart and the brain.
Fifth, reinforcing spleen and generating blood. The jujube extract and the extract of *Millettia reticuiata* are able to invigorate the spleen and warm the stomach, thus enhancing the growth and development.

The pharmacodynamic mechanisms of the pharmaceutical composition is specifically described combined with the components thereof. Of the pharmaceutical composition, the ginkgo flavone functions in promoting blood circulation and removing blood stasis; the jujube extract functions in tonifying the middle-jiao and replenishing qi, and nourishing blood to tranquilize mind; the extract of peel of *Trichosanthes kirilowii* functions in nourishing liver and kidney and alleviating depression to regulate qi; the saponin of *Codonopsis* functions in tonifying the middle-jiao and replenishing qi and strengthening spleen and lung; the saponin of American ginseng functions in invigorating qi and enriching yin; the saponin of *Radix astragalus* functions in replenishing qi to strengthen superficial body resistance, diuretic and dispel endotoxin; the hirudin functions in promoting blood circulation and freeing channels; the coenzyme Q10 is a strong oxidants functioning in improving the cardiac muscles and protecting the heart, and preventing the organism cells from being destructed by free radicals; the longan extract functions in invigorating qi and enriching yin, invigorating heart and spleen, and nourishing blood to tranquilize mind; the carthamin functions in promoting blood circulation and freeing channels, and dissipating the blood stasis and relieving pain; the extract of peach kernel functions in promoting blood circulation and removing blood stasis and alleviating constipation; the salvianolic acid functions in promoting blood circulation and removing blood stasis, tranquilize mind, cooling the blood and resolving carbuncle; the extract of *Ligusticum chuanxiong* functions in promoting qi and activating blood, expelling wind and alleviating pain; the saponin of *Panax notoginseng* functions in dissipating the blood stasis and promoting blood circulation, and alleviating swelling and pain.

It is demonstrated from experiments that after administration of the pharmaceutical composition by patients, the phospholipids, flavone, isoflavone, saponins, and compound enzymes of small molecular contained in the pharmaceutical composition quickly penetrate into atherosclerotic plaques and decompose lipid masses in the blood by breaking walls of lipid cells so as to gradually remove the lipids, decompose the plaques, expands and soften the blood vessels. As the expansion and the elasticity of the blood vessels are recovered, the resistance of the blood vessels is reduced, the flow velocity of the blood is facilitated, the pressure is reduced, the heartbeat and the breath become normal, an average increase of the proteoliposome is 33%, and an average decrease of triglyceride is 28%, a removal rate of the thrombus exceeds 98%. The blood supply for the blood vessels of the heart and brain, and hypertension and hyperlidemia almost disappear. After analyses of the components of the pharmaceutical composition, the processes of the above actions are as follows:

First, invigorating qi and promoting blood circulation

Qi is the power source of the body and the blood. Blood flow is maintained by the action of qi. When qi is deficient, the body is weak and uncomfortable, the blood flow is decreased, the breath is difficult, and the blood pressure increases. Thus, sufficient qi ensures the power of the blood circulation. Metabolism within human bodies is complicate biochemical processes involving different kinds of enzymes, which means that enzymes are catalysts for biochemical reactions within human body. The main component for hemocytolysis and blood vessel clearing is enzymes. Thus, by replenishing corresponding enzymes, the dysfunction of the endogenous enzyme system is recovered, function shortages are repaired, and blood lipids and thrombus coagulated in the blood vessels are removed, thus, the contradiction between the supply and the demand of the blood in the blood vessels is tackled, which is the basis and the primary task for removing the vascular obstacles.

The saponin of *Codonopsis,* the saponin of American ginseng, and the saponin of *Radix astragalus,* longan extract functions in nourishing yin and invigorating qi and promoting the blood circulation. The hirudin is indecomposable in vivo and is discharged as an active component via the kidney. The hirudin is easily acceptable by the organism, induces weak immunity, and does not affects the blood platelet, the fibrinogen level and the hemoglobin content, and is difficult to pass through the blood brain barrier. The hirudin is stable and the activity thereof is not changed by increasing the temperature or changing the pH value. An active component of hirudin is specific thrombin inhibitor, which is able to inhibit the free thrombin in the blood slurry as well as the thrombin combined with the blood clot, thus activating the injured heart and brain cells and being an important hemocytolysis component.

Second, soothing liver and clearing blood.

Under the action of the above enzyme, the pharmaceutical composition quickly penetrates into the plaque, the toxin microplates accumulated in the blood vessels are completely decomposed to sooth the blood vessels. However, the blood in the vessel still has large viscosity, the blood coagulation is high, and the blood flow is very slow, which results in increase of the blood pressure or even lockage of the blood vessel after long term. Thus, the blood must be purified by discharging the toxin outside the body. The blood vessel needs to be expanded and softened to recovery the expansion and elasticity of the blood vessels, facilitate the blood circulation, and reduce the blood pressure to remain the action of qi invigoration and blood circulation promotion.

In the above pharmaceutical composition, the carthamin, the extract of peach kernel, the salvianolic acid, and the extract of peel of *Trichosanthes kirilowii* assist in qi invigoration and blood circulation promotion. The treatment effect is enhanced, the broken brain nerve cells can be regenerated and connected, and therefore paralyses symptoms are removed. The extract of peel of *Trichosanthes kirilowii* functions in treating chest pain syndrome and resolving carbuncle, and assisting in removing the neurological disorder caused by the cardio-cerebrovascular disease.

Third, invigorating liver and kidney and filtering blood

Liver and kidney play important roles in the cardio-cerebrovascular diseases, as liver and kidney are able to regulate qi, promote operation of the essence and blood to convey the nutrient and oxygen to the brain along with the blood.

The astaxanthin.gastordin black sesamin functions in nourishing the essence and promoting the kidney and liver, expelling wind, promoting blood circulation and removing blood stasis. The astaxanthin is able to pass through the cell wall to directly remove the oxygen free radicals, enhance the regeneration ability, balance the body function, alleviate the accumulation of old cells and aging of the cardiovascular.

In addition, most patients suffered from cardio-cerebrovascular diseases has the problem of hypertension and dysfunction of the heart caused by kidney and liver deficiency. Particular in apoplexy and cerebral hemorrhages, the kidney and liver deficiency is more serious. Thus, the use of black sesamin in the treating the cardio-cerebrovascular diseases aims to promote liver and kidney and nourish essence. In addition, the black sesamin contains Vitamin E which is able to facilitate the cell division, remove the free radicals in the cells, and delay aging of the cells.

Fourth, enhancing heart for pumping blood

The ginkgo flavone, the coenzyme Q10 (liposoluble), the saponin of *Panax notoginseng,* and the extract of *Ligusticum chuanxiong* functions in antioxidation, strengthening the cardiac muscles, thus enhancing the activity of the heart and invigorating the human body, and assisting the primary components to enhance the efficacy of the pharmaceutical composition.

The ginkgo flavone primarily functions in inhibiting coagulation of platelet activating factors. Ginkgolides and bilobalide are able to protect the nerve cells of the central nerve system from being injured in ischemia conditions, promoting the blood circulation in the brain and the body. Furthermore, ginkgolides and bilobalide play anti-oxidant properties in the brain, the ocular retinal, and the cardiovascular system, thus preventing the aging of the brain functions, improving the blood flow of the brain, and nourishing the nerve system. It is known that ginkgo flavone is able to increase the blood flow in the cerebral blood vessels, improve the blood circulation in the cerebral blood vessels, prevent the angina pectoris, myocardial infarction, and the thrombus formation, thus being beneficial to coronary disease, angina pectoris, cerebral arteriosclerosis, senile dementia, and hypertension.

Coenzyme Q10 (liposoluble) is a strong antioxidant which is able to inhibit the formation and development of the atherosclerosis, stabilize the membrane, strengthen the heart, cure left ventricular hypertrophy, improve the metabolism of the cardiac muscles, and protect the cells of the organisms from being damaged by free radicals.

The extract of *Ligusticum chuanxiong* is able to promote qi and activate blood, and expel wind and alleviate pain. The saponin of *Panax notoginseng* functions in stopping bleeding, removing stasis, promoting the blood circulation, and improving the immunity, thus being effective to treat cardio-cerebrovascular diseases.

Fifth, reinforcing spleen and generating blood

It is considered in Chinese medicine that spleen is able to digest the food and distribute the nutrients to the whole body. Once the spleen is dysfunctional, the nutrients cannot be transported to different parts of the body, or the food is retained in the intestines, resulting in accumulation of toxins. Thus, spleen is the power source of the heart and provides the energy and nutrients for the heart beats. Once the heart is not well nourished, cardio-cerebrovascular diseases easily occur.

The jujube extract is able to tonify the middle-jiao and replenish qi, nourish blood to tranquilize mind. In addition, jujube polysaccharide in the jujube extract is able to complement activity, facilitate the propagation of the lymphocytes, improve the immunity of the body, inhibit the growth of the cancer cells, promote the formation of the leucocytes, reduce the level of the serum cholesterase, and protect the liver. The jujube extract also contains abundant vitamin C which is able to improve the swallow ability of monocytes in the body, protect the liver, reduce the level of cholesterol and the formation of calculus, promote the synthesis of proteins, and increase the content of the serum total proteins. Vitamin P contained in the jujube extract is able to protect the blood capillary, maintain the elasticity of the capillary wall, and is beneficial in anti-atherosclerosis, thus being able to prevent the cardio-cerebrovascular diseases.

The different components of the pharmaceutical composition do not just act in single steps, in fact, some of them acts in many processes. For example, the saponin of *Codonopsis* and the saponin of *Radix astragalus* play the main functions in the first step of invigorating qi and promoting blood circulation and assists in the fifth step of reinforcing spleen and generating blood; the extract of peel of *Trichosanthes kirilowii* functions in nourishing liver and kidney and alleviating depression to regulate qi, thus acts in the second step of soothing liver and clearing blood, the third step of invigorating liver and kidney and filtering blood, and the fourth step of enhancing heart for pumping blood.

The pharmaceutical composition is optionally formed by singly mixing the above components to for a mixture, or further preparing into different drug states by processing the mixture into tablets or powder. Thus, the pharmaceutical composition further comprises: 20-50 parts by weight of a modified starch; and preferably 30-40 parts by weight of the modified starch.

To reach better effect, preferably, a dose of the jujube extract is 50-80 parts by weight, and a dose of the longan extract t is 50-80 parts by weight.

To further optimize the effect of the pharmaceutical composition and reduce the side effect, purities of crucial components of the pharmaceutical composition are optimized, for example, a purity of the saponin of *Codonopsis* is equal to or larger than 80%, and a dose of the saponin of *Codonopsis* is 40-50 parts by weight.

For example, preferably, a purity of the saponin of American ginseng is larger than 80%, and a dose of the saponin of American ginseng is 60-70 parts by weight.

For example, preferably, a purity of the saponin of *Radix astragalus* is equal to or larger than 80%, and a dose of the saponin of *Radix astragalus* is 50-60 parts by weight.

For example, preferably, a purity of the hirudin is equal to or larger than 98%, and a dose of the hirudin is 25-50 parts by weight.

For example, preferably, the coenzyme Q10 is a liposoluble coenzyme Q10 having a purity of being equal to or larger than 80%, and a dose of the coenzyme Q10 is 4-8 parts by weight.

For example, preferably, a purity of the salvianolic acid is equal to or larger than 98%, and a dose of the salvianolic acid is 140-150 parts by weight.

For example, preferably, a purity of the ginkgo flavone is equal to or larger than 98%, and a dose of the ginkgo flavone is150-170 parts by weight; and a dose of the extract of peel of *Trichosanthes kirilowii* is 130-160 parts by weight.

For example, preferably, a purity of the carthamin is equal to or larger than 80%, and a dose of the carthamin is 130-150 parts by weight; and a dose of the extract of peach kernel is 100-120 parts by weight.

For example, preferably, a purity of the gastordin is equal to or larger than 98%, and a dose of the gastordin is 30-50 parts by weight, preferably 35-50 parts by weight; and a dose of the extract of *Ligusticum chuanxiong* is 50-70 parts by weight.

For example, preferably, a purity of the saponin of *Panax notoginseng* is equal to or larger than 98%, and a dose of the saponin of *Panax notoginseng* is 120-140 parts by weight.

For example, preferably, a purity of the black sesamin is equal to or larger than 98%, and a dose of the black sesamin is 30-50 parts by weight

For example, preferably, a purity of the astaxanthin is equal to or larger than 10%, and a dose of the astaxanthin is 30-50 parts by weight.

The above extracts are the extracts normally defined in the technical field. For example, one part by weight of the jujube extract is prepared by water extracting 10-30 parts by weight of decoction pieces of jujube. One part by weight of the extract of peel of *Trichosanthes kirilowii* is prepared by water extracting 10-30 parts by weight of decoction pieces of the peel of *Trichosanthes kirilowii.* One part by weight of the extract of peach kernel is prepared by water extracting 10-30 parts by weight of decoction pieces of the peach kernel. One part by weight of the extract of *Ligusticum chuanxiong* is prepared by water extracting 10-30 parts by weight of decoction pieces of *Ligusticum chuanxiong.* One part by weight of the extract of lycopodii herb is prepared by water extracting 10-30 parts by weight of decoction pieces of the lycopodii herb. One part by weight of the extract of *Fructus liquidambaris* is prepared by water extracting 10-30 parts by weight of decoction pieces of *Fructus liquidambaris.* One part by weight of the extract of *Eupolyphaga* is prepared by water extracting 10-30 parts by weight of decoction pieces of *Eupolyphaga.* One part by weight of the extract of *Scorpio* is prepared by water extracting 10-30 parts by weight of decoction pieces of *Scorpio.* One part by weight of the extract of stems or roots of *Fissistigma oldhamii* is prepared by water extracting 10-30 parts by weight of decoction pieces of stems or roots of *Fissistigma oldhamii.* One part by weight of the extract of *Lyeopodium complanatum* is prepared by water extracting 10-30 parts by weight of decoction pieces of *Lyeopodium complanatum.* One part by weight of the extract of *Lyeopodium complanatum* is prepared by water extracting 10-30 parts by weight of decoction pieces of *Lyeopodium complanatum.*

Raw materials of the pharmaceutical composition for use in treating cardio-cerebrovascular disease are prepared by supercritical biological extraction, the process of which is pure physical operation, no chemical or biochemical reactions occurs, the no additional chemicals are added into the pharmaceutical composition. And it is demonstrated from detection that the drug components have no side effects on liver, kidney, or other organs.

In another embodiment of the invention, a method for preparing the pharmaceutical composition is provided. The method comprises: 1) collecting raw materials for the pharmaceutical composition according to a formula, and uniformly mixing the raw materials in a dish to yield a mixture; 2) tableting the mixture, polishing resulting tablets, and sterilizing the tablets to yield a product, in which, sterilization is conducted at a temperature of 90-100°C and a time of 20-30 min.

The pharmaceutical composition of the invention is able to treat cardio-cerebrovascular diseases: hypertension, hyperlidemia, heart diseases, rheumatic heart diseases, both right and left atrial enlargement, serious ischemic heart diseases, angina pectoris, coronary arteriosclerosis, coronary atherosclerosis, shortness of breath, myocardial infarction, cerebral infarction, thromboembolisms, sequela of cerebrovascular disease, poststroke syndrome, apoplectic hemiplegia, and to prevent diabetes, senile dementia, tumors, and cancers.

A specification of the tablets of the pharmaceutical composition are deep brown or brown tablets, and each tablet has a net weight of 680 mg.

The tablets of the pharmaceutical composition are used as follows:
1) Patients suffered from hypertension, hyperlidemia, heart diseases, both right and left atrial enlargement, serious ischemic heart diseases, angina pectoris, coronary arteriosclerosis, coronary atherosclerosis, shortness of breath, myocardial infarction, cerebral infarction, thromboembolisms are administered with the tablets of the pharmaceutical composition for use in treating cardio-cerebrovascular disease for three times every day 40 min before diets, each time for two pieces, and each tablet has a net weight of 680 mg. The course of treatment lasts for 30 days.
2) Patients suffered from apoplectic hemiplegia, sequela of cerebrovascular disease, poststroke syndrome, senile dementia are also administered with a *Fructus liquidambaris* tablets (three times a day, two tablets for each time for first ten days and three tablets for each time later, and each tablet has a net weight of 550 mg) in addition to the tablets of the pharmaceutical composition for use in treating cardio-cerebrovascular disease of the invention (three times a day, two tablets for each time, and each tablets has a net weight of 680 mg). The tablets are administered 40 min before the diets and the course of treatment lasts for 30 days.

The *Fructus liquidambaris* tablet comprises the following components: 120-200 parts by weight of the extract of lycopodii herb, 150-230 parts by weight of the extract of *Fructus liquidambaris,* 120-160 parts by weight of extract of *Eupolyphaga,* 100-150 parts by weight of the extract of *Scorpion*, 130-180 parts by weight of the extract of *Caulis spatholobi,* 200-250 parts by weight of the extract of *Lyeopodium complanatum*, 50-150 parts by weight of the extract of stems or roots of *Fissistigma oldhamii,* and 20-50 parts by weight of the modified starch.

The pharmaceutical composition tablets have the following efficacies when being used in treating patients with hypertension, hyperlipemia, and insomnia:

Seven days after the administration, dizziness, headaches, and insomnia disappear, and the sleep quality is improved.

Fifteen days after the administration, indexes are decreased greatly, nightmare disappear, and the patients become vigorous.

Thirty days after the administration, the patients look good, shortness of breath, dizziness, nightmares disappear, the level of the serum lipid tends to be normal, the patients become vigorous.

After two courses of treatment, the blood pressure becomes normal, and the patients are cured and become vigorous. Patients with serious hypertension and hyperlipemia need six to eight courses of treatment to be completely cured.

Because the patients with hypertension always get used to the critical condition of the hypertension. Once the blood pressure sharply decreases, heartbeats facilitate and dizziness occur. Besides, general medicines for lowering the blood pressure are diuretics, which have the side effects of increment in the blood viscosity and formation of thrombus, thus aging the blood vessels after long term use.

The pharmaceutical composition tablets have the following efficacies when being used in treating patients with heart diseases:

Fifteen days after the administration, shortness of breath, cardio palmus, cardiac arrhythmia, premature beat, and dizziness disappear.

Thirty days after the administration, coronary disease, angina pectoris, heart failure, and myocardial ischemia are cured, and indexes of the heart become normal.

After two courses of treatment, the heartbeat become strong and the pulses are normal, the heart is strong and powerful. Patients with serious symptoms need six to eight courses of treatment to be completely cured.

The pharmaceutical composition tablets have the following efficacies when being used in treating patients with poststroke syndrome:

Fifteen days after the administration, dizziness and headaches disappear.

Thirty days after the administration, drooling disappears, limbs become warm and conscious, and patients are able to speak.

Forty-five days after the administration, patients are able to speak clearly, eat food, and do some basic activities by walking sticks.

After two courses of treatment, patients are able to speak clearly, eat food, and do some basic activities and outdoor activities.

After three courses of treatment, cerebral thrombosis, cerebral hemorrhage, and apoplectic hemiplegia are cured. Patients with serious symptoms need six to eight courses of treatment to be completely cured.

Thrombotic disease has high incidence, high complication rate, high misdiagnosis rate, high mortality rate, and thus safe and effective thrombolytic drugs is a hot topic of medical research. However, western medicines for treating thrombotic diseases has the efficacy leaving much to be improved and has sides effects. However, the pharmaceutical composition of the invention overcomes such disadvantages in treating thrombotic disease.

Hereinbelow, embodiments of the invention are specifically described. It should be noted that the embodiments aim to illustrate the invention and should not be deemed as restriction of the protection scope of the invention.

The purity of the ginkgo flavone is 98%; the purity of the saponin of *Codonopsis* is 80%; the purity of the saponin of American ginseng is 80%; the purity of the saponin of *Radix astragalus* is 80%; the purity of the hirudin is 98%; the purity of the carthamin is 80%; the purity of the gastordin is 98%; the purity of the salvianolic acid is 98%; the purity of the saponin of *Panax notoginseng* is 98%; the purity of the black sesamin is 98%; and the purity of the astaxanthin is equal to or larger than 10%.

The jujube extract, the extract of peel of *Trichosanthes kirilowii,* the longan extract, extract of peach kernel, the extract of *Ligusticum chuanxiong,* the extract of lycopodii herb, the extract of *Fructus liquidambaris,* the extract of *Eupolyphaga*, the extract of *Scorpio,* extract of *Millettia reticuiata,* the extract of *Lyeopodium complanatum*, and the extract of stems or roots of *Fissistigma oldhamii* are all purchased from Shaanxi Sen Fu Biological Technology Co., Ltd.

The purity of the coenzyme Q10 is 80%. In Examples 1-10, liposoluble coenzyme Q10 is adopted, while in Example 11 water soluble coenzyme Q10is adopted, and other formula is the same as Example 10. The formula for the tablets of *Fructus liquidambaris* of Example 11 is the same as that of Example 10.

**Table 1 Formula (unit: mg) of pharmaceutical composition of the invention**

| | Example 1 | Example 2 | Example 3 | Example 4 | Example 5 | Example 6 |
|---|---|---|---|---|---|---|
| Ginkgo flavone | 150 | 140 | 160 | 160 | 170 | 120 |
| Jujube extract | 80 | 70 | 50 | 80 | 60 | 100 |
| Extract of peel of *Trichosanthes kirilowii* | 130 | 130 | 150 | 150 | 160 | 140 |
| Saponin of *Codonopsis* | 50 | 50 | 50 | 50 | 50 | 55 |
| Saponin of American ginseng | 60 | 70 | 70 | 70 | 70 | 65 |
| Saponin of *Radix astragalus* | 50 | 60 | 50 | 50 | 50 | 45 |
| hirudin | 50 | 30 | 20 | 25 | 50 | 40 |
| Coenzyme Q10 | 4 | 4 | 4 | 4 | 4 | 7 |
| Longan extract | 70 | 50 | 70 | 80 | 80 | 63 |
| Modified starch | 30 | 30 | 30 | 30 | 30 | 40 |
| Carthamin | 130 | 130 | 130 | 150 | 130 | 140 |
| Extract of peach kernel | 100 | 120 | 110 | 100 | 100 | 120 |
| Gastordin | 50 | 50 | 40 | 50 | 36 | 40 |
| Salvianolic acid | 170 | 140 | 150 | 150 | 150 | 130 |
| Extract of *Ligusticum chuanxiong* | 60 | 50 | 60 | 55 | 70 | 45 |
| Saponin of *Panax notoginseng* | 140 | 140 | 140 | 140 | 140 | 130 |
| Black sesamin | 50 | 50 | 50 | 0 | 30 | 40 |
| Astaxanthin | 36 | 36 | 36 | 36 | 0 | 40 |
| Total | 1360 | 1350 | 1370 | 1380 | 1380 | 1360 |

**Table 2 Formula (unit: mg) of pharmaceutical composition of the invention**

| | Example 7 | Example 8 | Example 9 | Example 10 | Example 11 |
|---|---|---|---|---|---|
| Ginkgo flavone | 155 | 165 | 150 | 165 | 165 |
| Jujube extract | 75 | 65 | 80 | 55 | 55 |
| Extract of peel of Trichosanthes kirilowii | 140 | 145 | 145 | 155 | 155 |
| Saponin of Codonopsis | 40 | 45 | 55 | 45 | 45 |
| Saponin of American ginseng | 60 | 65 | 65 | 65 | 65 |
| Saponin of *Radix astragalus* | 55 | 50 | 60 | 55 | 55 |
| Hirudin | 25 | 20 | 35 | 45 | 45 |
| Coenzyme Q10 | 6 | 8 | 9 | 4 | 4 |
| Longan extract | 64 | 52 | 61 | 75 | 75 |
| Modified starch | 35 | 40 | 35 | 40 | 40 |
| Carthamin | 135 | 145 | 135 | 145 | 145 |
| Extract of peach kernel | 105 | 115 | 105 | 106 | 106 |
| Gastordin | 45 | 35 | 50 | 35 | 35 |
| Salvianolic acid | 145 | 135 | 140 | 140 | 140 |
| Extract of Ligusticum chuanxiong | 65 | 55 | 65 | 60 | 60 |
| Saponin of *Panax notoginseng* | 120 | 125 | 125 | 135 | 135 |
| Black sesamin | 45 | 50 | 0 | 35 | 35 |
| Astaxanthin | 45 | 45 | 45 | 0 | 0 |
| Total | 1360 | 1360 | 1360 | 1360 | 1360 |

A method for preparing the pharmaceutical composition in the above examples is conducted as follows:
1) Raw materials of ginkgo flavone, jujube extract, extract of peel of *Trichosanthes kirilowii,* saponin of *Codonopsis,* saponin of American ginseng, saponin of *Radix astragalus,* hirudin, coenzyme Q10 (liposoluble), longan extract, modified starch, carthamin, extract of peach kernel, gastordin, salvianolic acid, extract of *Ligusticum chuanxiong,* saponin of *Panax notoginseng,* black sesamin, astaxanthin in powder states were collected according to a formula and collected.
2) The raw materials are mixed in a dish for 20 min to yield a mixture.
3) The mixture is tableted to yield tablets, and each tablet has a net weight of 680 mg, the tablets are then polished, and sterilized for 20 min at 100°C.

The efficacy of the pharmaceutical composition in the above embodiment are as follows:
It is indicated from the clinical results that patients suffered from the disease within one year become normal in seven days, and patients suffered from the disease more than one year also have effect after taking the pharmaceutical compositions of the invention of several stages.
Time:
From Feb. 10, 2012 to Mar. 10, 2013.
Experiment site:
Academy of Military Medical Sciences, No.43, Taiping Road, Haidian District, Beijing

Experiment purpose:
To detect the influence of the pharmaceutical composition of the organs of human body and the thrombolytic efficacy of the pharmaceutical composition.

Experiment methods:
Group A: fifteen hypertension patients, administered with tablets of Example 1 three times daily, each time with two tablets 40 min before diets, each tablet with a net weight of 680 mg, the administration lasts for eight treatment course (30 days for one treatment course).
Group B: twenty-five patients suffered from coronary disease, administered with tablets of Example 2 three times daily, each time with two tablets 40 min before diets, each tablet with a net weight of 680 mg, the administration lasts for eight treatment course (30 days for one treatment course).
Group C: sixty hyperlipemia patients, divided into four groups with each group having fifteen patients and respectively administered with tablets of Example 3, Example 4, Example 5, Example 6 three times daily, each time with two tablets 40 min before diets, each tablet with a net weight of 680 mg, the administration lasts for eight treatment course (30 days for one treatment course).
Group D: sixty paralyzed patients, divided into six groups with each group having ten patients and respectively administered with tablets of Example 7, Example 8, Example 9, Example 10 (three times daily, each time with two tablets, each tablet with a net weight of 680 mg) and at the same time administered with the *Fructus liquidambaris* tablets (three times daily for the first ten days and then twice daily, each time with two tablets, each tablet with a net weight of 550 mg). The administrations last for eight treatment course (30 days for one treatment course).
Another group is adopted as a control group and just administered with the tablets of Example 7 (three times daily, each time with two tablets, each tablet with a net weight of 680 mg).
Formula of the *Fructus liquidambaris* tablets of Group D is listed in Table 3:

**Table 3**

| | Example 7 | Example 8 | Example 9 | Example 10 | Example 11 |
|---|---|---|---|---|---|
| Extract of lycopodii herb | 165 | 125 | 155 | 170 | 170 |
| Extract of *Fructus liquidambaris* | 180 | 220 | 170 | 185 | 185 |
| Extract of *Eupolyphaga* | 125 | 130 | 155 | 145 | 145 |
| Extract of *Scorpio* | 115 | 125 | 110 | 105 | 105 |
| Extract of *Millettia reticuiata* | 145 | 135 | 155 | 165 | 165 |
| Extract of *Lyeopodium complanatum* | 250 | 245 | 235 | 210 | 210 |
| Extract of stems or roots of *Fissistigma oldhamii* | 80 | 85 | 95 | 75 | 75 |
| Modified starch | 40 | 35 | 25 | 45 | 45 |
| Total | 1100 | 1100 | 1100 | 1100 | 1100 |

The method for preparing the *Fructus liquidambaris* tablets of Group D is the same as that for preparing the pharmaceutical composition of the invention.
Group E: thrombolysis comparison, patients of group F and group G are respectively administered with the pharmaceutical composition of the invention and western medicines, and efficacy of the two groups is compared. The patients of the two groups have no significant difference in age, sex, position of thrombus, course of disease, and clinical manifestations.

### Comparison of thrombolysis

Group F: administered with the pharmaceutical composition of the invention, fifteen patients are administered with tablets (twice tablets three times daily, each tablet has a net weight of 680 mg) of Example 6 for 30 days, and it is known from B-ultrasonography that twelve patients have the atrial thrombus completely disappear, and three patients have the atrial thrombus partially disappear.

Group G: Control group administered with western medicines. Fifteen patients are treated with anticoagulation for 30 days. It is known from the B-ultrasonography that seven patients have the thrombus completely disappear, six patients have the thrombus partially disappear, and two patients have no efficacy.

### Experiment results:

Group A: patients with hypertension have the following symptoms, 15 patients have diastolic pressure exceeding 180 mmHg and systolic pressure exceeding 110 mmHg, dizziness, syrigmus, choking sensation in chest, shortness of breath, insomnia, and partial obstruction of blood vessel represented by transcranial Doppler.

After seven days administrated of the pharmaceutical composition of the invention, the dizziness, headache, and insomnia disappear, and the sleep quality is improved.

Fifteen days after the administration, indexes are decreased greatly, nightmare disappear, and the patients become vigorous.

Thirty days after the administration, the patients look good, shortness of breath, dizziness, nightmares disappear, the level of the serum lipid tends to be normal, the patients become vigorous.

After three courses of treatment, the blood pressure becomes normal, and the patients are cured and become vigorous.

After eight courses of treatment by using the pharmaceutical composition of the invention, thirteen patients have the diastolic pressure lowered to 140 mmHg below and the systolic pressure reduced to approximately 100 mmHg. The main symptoms disappear, no abnormal symptoms is indicated by cranial Doppler, and the blood supply of the heart and brain are normal indicated by the electrocardiogram. One patient has the diastolic pressure of 150 mmHg and the systolic pressure of 100 mmHg, and the main symptoms disappear, r, no abnormal symptoms is indicated by cranial Doppler, and the blood supply of the heart and brain are normal indicated by the electrocardiogram. The pharmaceutical composition does not work in one patient.

Group B: patients are suffered from coronary diseases. 25 patients have symptoms of palpitation, shortness of breath, irregular pain in chest, weakness, and serious insufficiency of blood supply.

Fifteen days after the administration, shortness of breath, cardio palmus, cardiac arrhythmia, premature beat, and dizziness disappear.

Thirty days after the administration, coronary disease, angina pectoris, heart failure, and myocardial ischemia are cured, and indexes of the heart become normal.

After two courses of treatment, the heartbeat become strong and the pulses are normal, the heart is strong and powerful. Patients with serious symptoms need six to eight courses of treatment to be completely cured.

After three courses of treatment using the tablets of the invention, 22 of 25 patients have their symptoms improved. For 16 patients have electrocardiographic reexamination, 14 patients have V5T improved. For 13 patients that have lowered S-T section before administration, 12 patients are improved, and only one patient has no efficacy. An average amplitude of the V5T wave before administration is 0.96 mm and increase to 2.7 mm after administration. An average drop of the S-T section is 0.84 mm before administration and 0.27 mm after administration. For one patient who has anterior myocardial infraction and inverted T wave and V2-V4QS wave detected by electrocardiogram, the symptoms of the patients are obviously improved after two courses of treatment, the V2-V4Q wave disappears, and the V5T wave faces upwards. Another patient who has angina pectoris and static sinus and flat V5T wave, after eight courses of treatment, the T wave become normal and the static sinus disappear.

The most obvious change is found in one case before the medication in February, the old anterior myocardial infarction, electrocardiogram V2-V4QS wave, T wave inversion, medication after two courses of symptoms improved significantly, V2-V4Q wave disappeared, V5T wave up; The other 1 case of the original angina pectoris and sinus static, V5T wave flat, the use of the tablet of the invention after 8 courses, T wave returned to normal sinus static disappear.

Group C: after eight courses of treatment by using the tablets of the pharmaceutical composition of the invention, 59 patients have their symptoms disappear and index of the blood lipid become normal. One patient administered with the pharmaceutical composition of Example 4 has their symptoms alleviated but the index of the blood lipid still higher than a normal level.

Group D: 60 patients have symptoms of unconsciousness and paralysis.

After administrated with the pharmaceutical compositions, efficacies are listed in Table 4:

**Table 4**

| | Example 7 | Example 8 | Example 9 | Example 10 | Example 11 | Control group |
|---|---|---|---|---|---|---|
| Experim ent patients | Ten | Ten | Ten | Ten | Ten | Ten |
| Main symptoms | Unconsciou sness and paralysis | Unconsciou sness and paralysis | Unconsciou sness and paralysis | Unconsciou sness and paralysis | Unconsciou sness and paralysis | Unconsciou sness and paralysis |
| After 15 days of administration | No dizziness headache | No ordizziness headache | No ordizziness headache | No ordizziness headache | No ordizziness headache | No ordizziness or headache |
| After 30 days of administration | No drooling, attempting to speak, | No drooling, attempting to speak, | No drooling, attempting to speak, | No drooling, attempting to speak, | No drooling, attempting to speak, | No drooling, attempting to speak, |
| | limbs become warm and conscious | limbs become warm and conscious | limbs become warm and conscious | limbs become warm and conscious | limbs become warm and conscious | limbs become warm and conscious |
| After three courses of treatment | Able to speak clearly, eat food, and do basic activities assistance | Able to speak clearly, eat food, and do basic by activities assistance | Able to speak clearly, eat food, and do basic by activities assistance | Able to speak clearly, eat food, and do basic by activities assistance | Able to speak clearly, eat food, and do basic by activities assistance | Able to speak clearly, eat food, and do basic by activities by assistance |
| After five courses of treatment | Six patients can do some outdoor activities and basic housework; and four patients become conscious and can speak, and the limbs | Five patients can do some outdoor activities and basic housework | Five patients can do some outdoor activities and basic housework | Four patients can do some outdoor activities and basic housework | Two patients can do some outdoor activities and basic housework | One patients can do some outdoor activities and basic housework |
| | are movable | | | | | |
| After eight courses of treatment | Six patients can do some outdoor activities and basic housework; and four patients become conscious and can speak, and the limbs are movable | Six patients can do some outdoor activities and basic housework; and two patients become conscious and can speak, and the limbs are movable | Six patients can do some outdoor activities and basic housework; and one patient become conscious and can speak, and the limbs are movable | Five patients can do some outdoor activities and basic housework; and one patients become conscious and can speak, and the limbs are movable | Two patients can do some outdoor activities and basic housework; and one patient become conscious and can speak, and the limbs are movable | One patient can do some outdoor activities and basic housework |

### Comparison of thrombolysis

Group F: administered with the pharmaceutical composition of the invention, fifteen patients are administered with tablets (twice tablets three times daily, each tablet has a net weight of 680 mg) of Example 6 for 30 days, and it is known from B-ultrasonography that twelve patients have the atrial thrombus completely disappear, and three patients have the atrial thrombus partially disappear.

Group G: Control group administered with western medicines. Fifteen patients are treated with anticoagulation for 30 days. It is known from the B-ultrasonography that seven patients have the thrombus completely disappear, six patients have the thrombus partially disappear, and two patients have no efficacy.

Complications in thrombolysis: fifteen patients of Group F have no bleeding symptoms, and three patients have slight gastrointestinal reactions and the gastrointestinal reactions disappear after symptomatic treatment. Patients of the group F are performed with the blood coagulation test, results of which all fall within the normal ranges. Group G is a control group administered with western medicine, six patients have gingival bleeding and subcutaneous ecchymosis, and two patients have lower limb ulceration. And it is known from the laboratory hematuria that in intravenous administration of heparin, oral administration of warfarin, and intravenous injection of urokinase of Group G, APTT, fibrinogen, anticoagulant III, and D-dimer must be regularly detected, and it is known that the results were significantly changed. The pharmaceutical composition of the invention is effect and safe and does not require blood test when being used in treating cardio-cerebrovascular diseases

At present, the clinical anti-thrombotic drugs are divided into anticoagulants, anti-platelet aggregation drugs, and thrombolytic drugs:
A. The anticoagulants are a kind of drugs that interference blood coagulation factors and inhibits the blood coagulation, and are mainly used in preventing and treating the thromboembolic disease.
B. The anti-platelet aggregation drugs are divided into three generations: aspirin is the first generation, ticlopidine is the second generation, and platelet glycoprotein II b / III a receptor antagonist is the third generation. Among them, the platelet glycoprotein II b / III a receptor antagonist is an important drug in anti-platelet therapy.
C. Fibrin formed in blood coagulation can be decomposed into soluble products from the arginine-lysine bonds under the action of plasmin, thus realizing thrombolysis. The fibrinolytic drugs activate the plasmin and facilitates the thrombolysis, which are also

The pharmaceutical composition of the invention is different from the above anticoagulant thrombolytic drugs for treating cardio-cerebrovascular diseases in the following respects:

First, differences in components of the raw materials. The conventional drugs for treating cardio-cerebrovascular diseases employs nattokinases as the raw materials, however, the nattokinases easily induce symptoms of gout and lupus erythematosus. Thus, gout patients are inhibited from taking the anti-coagulants and thrombolytic drugs. The drug components of the invention do not contain heparin, ticlopidine, streptokinase, urokinase, or nattokinase, thus having no side effects on human body.

Second, the anti-coagulants and thrombolytic drugs containing heparin, ticlopidine, streptokinase, and urokinase easily lead to bleeding of the patients.

The pharmaceutical composition of the invention contains the natural hirudin component, which is an important active component and a specific thrombin inhibitor that is able to not only inhibit the free thrombins in the serum but also effectively preventing the thrombin from combining with the blood clotting. Besides, hirudin is almost not metabolized by the liver and is discharged in the urine in the form of the original form or a derivative, which also has the anti-thrombin function. It is indicated from the clinical study that hirudin has good efficacy in treating acute coronary syndrome (ACS) and the efficacy thereof is more significant than the heparin and has obvious dose-effect relationship. When adopting hirudin to treat the thrombocytopenia caused by heparin, 14.4 hrs after the treatment, 88.7% of the patients have their blood platelets recovered and normal, and bleeding only occurs in a dose much larger than the required dose for anti-coagulation.

Third, the action principles are different. The conventional drug for treating cardio-cerebrovascular diseases mainly focus on hemolysis and blood circulation promotion. While the pharmaceutical composition of the invention focus on invigorating qi, nourishing blood, and thrombolysis. By invigorating qi, the heart pumping is improved, and the blood circulation is promoted, thus, the cardio-cerebrovascular diseases is completely cured, the blood pressure is recovered and the heart becomes normal.

Fourth, the doses are different. The pharmaceutical composition of the invention is administered three times daily with two tablets for each time, while the conventional drug is administered with 4-5 pills for each time.

Fifth, the efficacies are different. The pharmaceutical composition of the invention is quickly penetrated and absorbed, and works in a short time, in the meanwhile, it also has functions of regulating endothelial cell, inhibiting angiospasm, and decreasing the level of the cell factors causing inflammations and thrombus. The pharmaceutical composition of the invention has no influence on the formation of the blood platelets, and does not induce the decrease of the blood platelets and thrombus formation like heparin. Thus, the pharmaceutical composition has good effect in improving ASO and TAO. However, the conventional drugs for treating cardio-cerebrovascular diseases are slowly penetrated, absorbed, and it takes a long time to work efficiently. The conventional drugs are unable to regulate the cell inflammatory factors but affect the formation of the blood platelets.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A pharmaceutical composition for use in treating cardio-cerebrovascular disease, the pharmaceutical composition comprising: 100-170 parts by weight of a ginkgo flavone, 40-100 parts by weight of a jujube extract, 100-200 parts by weight of an extract of peel of *Trichosanthes kirilowii,* 30-60 parts by weight of a saponin of *Codonopsis,* 50-100 parts by weight of a saponin of American ginseng, 40-70 parts by weight of a saponin of *Radix astragalu,* 20-50 parts by weight of hirudin, 3-10 parts by weight of a coenzyme Q10, 40-100 parts by weight of a longan extract, 20-50 parts by weight of a modified starch, 100-150 parts by weight of a carthamin, 100-150 parts by weight of an extract of peach kernel, 30-50 parts by weight of a gastordin, 120-200 parts by weight of a salvianolic acid, 40-90 parts by weight of an extract of *Ligusticum chuanxiong,* 100-150 parts by weight of a saponin of *Panax notoginseng,* 0-50 parts by weight of a black sesamin, 0-50 parts by weight of an astaxanthin.

2. The pharmaceutical composition of claim 1, **characterized in that** the ginkgo flavone has a purity of equal to or larger than 98%, and a dose of the ginkgo flavone is 150-170 parts by weight; a dose of the jujube extract is 50-80 parts by weight; and a dose of the extract of the peel of *Trichosanthes kirilowii* is 130-160 parts by weight.

3. The pharmaceutical composition of claim 1, **characterized in that** a purity of the saponin of *Codonopsis* is equal to or larger than 80%, and a dose of the saponin of *Codonopsis* is 40-50 parts by weight; a purity of the saponin of American ginseng is larger than 80%, and a dose of the saponin of American ginseng is 60-70 parts by weight; a purity of the saponin of *Radix astragalus* is equal to or larger than 80%, and a dose of the saponin of American ginseng is 50-60 parts by weight.

4. The pharmaceutical composition of claim 1, **characterized in that** a purity of the hirudin is equal to or larger than 98%, and a dose of the hirudin is 25-50 parts by weight; a purity of the coenzyme Q10 is equal to or larger than 80%, and a dose of the coenzyme Q10 is 4-8 parts by weight; and a dose of the longan extract is 50-80 parts by weight.

5. The pharmaceutical composition of claim 1, **characterized in that** a dose of the modified starch is 30-40 parts by weight; a purity of the carthamin is equal to or larger than 80%, and a dose of the carthamin is 130-150 parts by weight; and a dose of the extract of peach kernel is 100-120 parts by weight.

6. The pharmaceutical composition of claim 1, **characterized in that** a purity of the gastordin is equal to or larger than 98%, and a dose of the gastordin is 35-50 parts by weight; a purity of the salvianolic acid is equal to or larger than 98%, and a dose of the salvianolic acid is 140-150 parts by weight; and a dose of the extract of *Ligusticum chuanxiong* is 50-70 parts by weight.

7. The pharmaceutical composition of claim 1, **characterized in that** a purity of the saponin of *Panax notoginseng* is equal to or larger than 98%, and a dose of the saponin of *Panax notoginseng* is 120-140 parts by weight.

8. The pharmaceutical composition of claim 1, **characterized in that** a purity of the black sesamin is equal to or larger than 98%, and a dose of the black sesamin is 30-50 parts by weight.

9. The pharmaceutical composition of claim 1, **characterized in that** a purity of the astaxanthin is equal to or larger than 10%, and a dose of the astaxanthin is 30-50 parts by weight.

10. A method for preparing the pharmaceutical composition of any of claims 1-9, the method comprising: 1) collecting raw materials for the pharmaceutical composition according to a formula, and uniformly mixing the raw materials in a dish to yield a mixture; 2) tableting the mixture, polishing resulting tablets, and sterilizing the tablets to yield a product; wherein sterilization is conducted at a temperature of 90-100°C and a time of 20-30 min.

11. A pharmaceutical composition for use in treating cardio-cerebrovascular disease, the pharmaceutical composition comprising: 100-170 parts by weight of a ginkgo flavone, 40-100 parts by weight of a jujube extract, 100-200 parts by weight of an extract of peel of *Trichosanthes kirilowii,* 30-60 parts by weight of a saponin of *Codonopsis,* 50-100 parts by weight of a saponin of American ginseng, 40-70 parts by weight of a saponin of *Radix astragalu,* 20-50 parts by weight of hirudin, 3-10 parts by weight of a coenzyme Q10, 40-100 parts by weight of a longan extract, 100-150 parts by weight of a carthamin, 100-150 parts by weight of an extract of peach kernel, 0-50 parts by weight of a gastordin, 120-200 parts by weight of a salvianolic acid, 40-90 parts by weight of an extract of *Ligusticum chuanxiong,* 100-150 parts by weight of a saponin of *Panax notoginseng*, 0-50 parts by weight of a black sesamin, and 0-50 parts by weight of an astaxanthin.

12. The pharmaceutical composition of claim 11, further comprising 20-50 parts by weight of a modified starch; and preferably 30-40 parts by weight of the modified starch.

13. The pharmaceutical composition of claim 11, **characterized in that** a dose of the jujube extract is 50-80 parts by weight.

14. The pharmaceutical composition of claim 11, **characterized in that** a purity of the saponin of *Codonopsis* is equal to or larger than 80%, and a dose of the saponin of *Codonopsis* is 40-50 parts by weight.

15. The pharmaceutical composition of claim 11, **characterized in that** a purity of the saponin of American ginseng is larger than 80%, and a dose of the saponin of American ginseng is 60-70 parts by weight.

16. The pharmaceutical composition of claim 11, **characterized in that** a purity of the saponin of *Radix astragalus* is equal to or larger than 80%, and a dose of the saponin of *Radix astragalus* is 50-60 parts by weight.

17. The pharmaceutical composition of claim 11, **characterized in that** a purity of the hirudin is equal to or larger than 98%, and a dose of the hirudin is 25-50 parts by weight.

18. The pharmaceutical composition of claim 11, **characterized in that** the coenzyme Q10 is a liposoluble coenzyme Q10 having a purity of being equal to or larger than 80%, and a dose of the coenzyme Q10 is 4-8 parts by weight.

19. The pharmaceutical composition of claim 11, **characterized in that** a dose of the longan extract is 50-80 parts by weight.

20. The pharmaceutical composition of claim 11, **characterized in that** a purity of the salvianolic acid is equal to or larger than 98%, and a dose of the salvianolic acid is 140-150 parts by weight.

21. The pharmaceutical composition of claim 11, **characterized in that** a purity of the ginkgo flavone is equal to or larger than 98%, and a dose of the ginkgo flavone is 150-170 parts by weight; and a dose of the extract of peel of *Trichosanthes kirilowii* is 130-160 parts by weight.

22. The pharmaceutical composition of claim 11, **characterized in that** a purity of the carthamin is equal to or larger than 80%, and a dose of the carthamin is 130-150 parts by weight; and a dose of the extract of peach kernel is 100-120 parts by weight.

23. The pharmaceutical composition of claim 11, **characterized in that** a purity of the gastordin is equal to or larger than 98%, and a dose of the gastordin is 30-50 parts by weight, preferably 35-50 parts by weight; and a dose of the extract of *Ligusticum chuanxiong* is 50-70 parts by weight.

24. The pharmaceutical composition of claim 11, **characterized in that** a purity of the saponin of *Panax notoginseng* is equal to or larger than 98%, and a dose of the saponin of *Panax notoginseng* is 120-140 parts by weight.

25. The pharmaceutical composition of claim 11, **characterized in that** a purity of the black sesamin is equal to or larger than 98%, and a dose of the black sesamin is 30-50 parts by weight.

26. The pharmaceutical composition of claim 11, **characterized in that** a purity of the astaxanthin is equal to or larger than 10%, and a dose of the astaxanthin is 30-50 parts by weight.

27. The pharmaceutical composition of claim 11, **characterized in that** the composition comprises: 150 parts by weight of the ginkgo flavone, 80 parts by weight of the jujube extract, 130 parts by weight of the extract of peel of *Trichosanthes kirilowii,* 50 parts by weight of the saponin of *Codonopsis,* 60 parts by weight of the saponin of American ginseng, 50 parts by weight of the saponin of *Radix astragalu,* 50 parts by weight of hirudin, 4 parts by weight of the coenzyme Q10, 70 parts by weight of the longan extract, 130 parts by weight of the carthamin, 100 parts by weight of the extract of peach kernel, 50 parts by weight of the gastordin, 170 parts by weight of the salvianolic acid, 60 parts by weight of the extract of *Ligusticum chuanxiong,* 140 parts by weight of saponin of *Panax notoginseng,* 50 parts by weight of the black sesamin, and 36 parts by weight of the astaxanthin.

28. A method for preparing the pharmaceutical composition of any of claims 11-27, the method comprising: 1) collecting raw materials for the pharmaceutical composition according to a formula, and uniformly mixing the raw materials in a dish to yield a mixture; 2) tableting the mixture, polishing resulting tablets, and sterilizing the tablets to yield a product; wherein sterilization is conducted at a temperature of 90-100°C and a time of 20-30 min.

29. Use of the pharmaceutical composition of any of claims 11-27 for treating hypertension, hyperlidemia, heart diseases, rheumatic heart diseases, both right and left atrial enlargement, serious ischemic heart diseases, angina pectoris, coronary arteriosclerosis, coronary atherosclerosis, shortness of breath, myocardial infarction, cerebral infarction, thromboembolisms, sequela of cerebrovascular disease, poststroke syndrome, or apoplectic hemiplegia.

30. The use of claim 29, **characterized in that** the pharmaceutical composition is used combining with use of a pharmaceutical composition of *Fructus liquidambaris.*
